Europäisches Patentamt

European Patent Office

Office européen des brevets

(11) Veröffentlichungsnummer: **0 267 355 B1**

# (12) EUROPÄISCHE PATENTSCHRIFT

(45) Veröffentlichungstag der Patentschrift: **18.09.91**

(51) Int. Cl.⁵: **G01N 33/68**, G01N 33/577

(21) Anmeldenummer: **87104557.1**

(22) Anmeldetag: **27.03.87**

(54) **Verfahren zum Identifizieren gewebstypischer, unlöslicher Cytoskelettproteine.**

(30) Priorität: **10.11.86 LU 86652**
**10.11.86 LU 86655**

(43) Veröffentlichungstag der Anmeldung:
**18.05.88 Patentblatt 88/20**

(45) Bekanntmachung des Hinweises auf die
Patenterteilung:
**18.09.91 Patentblatt 91/38**

(84) Benannte Vertragsstaaten:
**AT BE CH DE FR GB IT LI NL SE**

(56) Entgegenhaltungen:
**EP-A- 0 163 304**

**CHEMICAL ABSTRACTS, Band 101, Nr.5, 30
Juli 1984, Columbus, OH (US); S.I.DANTO et
al., Seite 395, Nr.37046z**

**CHEMICAL ABSTRACTS, Band 96, Nr.1, 04
Jänner 1982, Columbus, OH (US);
G.GABBIANI et al., Seite 286, Nr.3170y**

(73) Patentinhaber: **PROGEN Biotechnik GmbH
Im Neuenheimer Feld 519
W-6900 Heidelberg(DE)**

(72) Erfinder: **Bruder, Gerda, Dr.
Bergheimer Str. 110A
W-6900 Heidelberg(DE)**
Erfinder: **Franke, Werner Wilhelm, Prof.Dr.
Landfriedstr. 6
W-6900 Heidelberg(DE)**

(74) Vertreter: **Hach, Hans Karl, Dr.
Tarunstrasse 23
W-6950 Mosbach-Waldstadt(DE)**

**Beschreibung**

Die Erfindung betrifft ein Verfahren zum Identifizieren gewebstypischer Proteine unlöslicher Strukturen mit spezifischen Antikörpern, deren Antigene gewebstypische Abschnitte des Proteins darstellen.

Die mit der Plasmamembran assoziierten Proteine cytoplasmatischer Plaque-Strukturen sind durch hohe Unlöslichkeit in biochemischen Puffern ausgezeichnet; auch hier kommen Zelltyp-spezifische Hauptproteine vor. Besonders gut untersucht ist die Zusammensetzung der Desmosomen-Plaques, deren Hauptproteine Desmoglein, Plakoglobin und die Desmoplakine I und II histodiagnostisch typisch für Epithelien und epitheliale Tumoren wie auch Meningen und Meningiome sind. Das Auftreten von Fragmenten dieser Hauptproteine in Körperflüssigkeiten stellt einen geeigneten Indikator für Zelläsionen und destruktive Prozesse in epithelialen und meningialen Gewebetypen dar. Es ist also wünschenswert, solche Proteine zu identifizieren.

Aus EP-A-0 163 304 ist es bekannt, mit spezifischen Antikörpern histodiagnostisch Intermediärfilamentproteine zu identifizieren. Intermediärfilamentproteine sind Bestandteile des Cytoskeletts und jeweils typisch für eine Gewebeklasse. Eine solche Identifizierung ermöglicht es, Intermediärfilamentproteine von Metastasen oder Gewebsläsionen dem Ursprungsgewebe beziehungsweise dem Primärtumor zuzuordnen.

Histologische Untersuchungen sind umständlich und in ihrer Anwendung beschränkt und führen nur zum Erfolg, wenn die betreffende Gewebeart in vitro identifizierbar oder für eine Untersuchung zugänglich ist.

Aufgabe der Erfindung ist es, ein Verfahren der eingangs genannten Art so auszugestalten, daß es in der Praxis leicht und auch vielfältig anzuwenden ist und eine möglichst große Entdeckungschance bietet.

Die Erfindung ist dadurch gekennzeichnet, daß Fragmente, die das Antigen tragen und die den charakteristischen Abschnitten entsprechen, in Lösung gebracht und dann immunologisch bestimmt und quantifiziert werden.

Die gewebstypischen Proteine unlöslicher Strukturen sind Intermediärfilamentproteine. Bei Intermediärfilamentproteinen enthalten die Fragmente die alpha-helikalen Mittelstücke oder Teile davon. Solche Fragmente werden alpha-helikale Mittelstück-Fragmente genannt. Alle alpha- helikalen Mittelstück-Fragmente die hier gemeint sind, tragen das Antigen und entsprechen den oben genanntencharakteristischen Abschnitten.

Die Erfindung macht sich dabei die folgenden Erkenntnisse aus der Zellbiologie zunutze:

Intermediärfilament(IF)-Proteine, die als Bestandteile des Cytoskeletts zu den unlöslichen Zellbestandteilen gehören, sind durch hohe Stabilität ausgezeichnete Proteine von großer Zelltypspezifität. Die Zuordnung ergibt sich aus der nachfolgenden Tabelle I.

TABELLE 1

| Gewebe | Intermediärfilamentprotein |
|---|---|
| Muskel- | Desmin |
| Neuronal- | Neurofilamentproteine (NF-L, NF-M, NF-H) |
| Mesenchymal- | Vimentin |
| Astrozyten | Gliafilament |
| Epithel- | Cytokeratine |

Das Nachweisverfahren geschieht mit Hilfe eines Sandwich-ELISA, der für jeden Filament-Typ aus jeweils zwei spezifischen Antikörpern aufgebaut ist.

Unter anderem zum Nachweis von Metastasen ist es wünschenswert, unterschiedliche Zelltypen in einem Gewebe zu identifizieren. Dies kann dadurch geschehen, daß aus Zellgewebe ein Homogenat gebildet wird, daß auf das Homogenat abbauend eingewirkt wird, bis aus eventuell dann enthaltenen Intermediärfilamentproteinen die alphahelikale Mittelstückfragmente freigesetzt sind, daß dann die lösliche Fraktion abgetrennt wird und, daß dann die in der löslichen Fraktion enthaltenen alpha-helikalen Mittelstück-Fragmente in löslicher Phase immunologisch identifiziert werden.

Aufgabe einer Weiterbildung ist es, über das Ausmaß der Metastasierung eine Aussage zu machen. Diese Aufgabe wird dadurch gelöst, daß verschiedene Intermediärfilamentproteine eines Zellhomogenats identifiziert und mengenmäßig zueinander ins Verhältnis gesetzt werden.

Es gibt verschiedene Cytokeratine (CK NR 1 - 19), deren Expressionsmuster verschiedenen Epithelgeweben zugeordnet sind. Deshalb wird vorzugsweise bei Cytokeratinen die Art des jeweils zugeordneten Epithelgewebes anhand der relativen Anteile der identifizierten Cytokeratine bestimmt und quantifiziert.

Auch für diese Zuordnung liegen hinreichende Charakteristika in den alpha-helikalen Mittelstücken vor.

Um sicherzustellen, daß die alpha-helikalen Mittelstücke beim Abbau nicht so weit mit zerstört werden, daß die Identifikation behindert wird, empfiehlt es sich, daß diese nur so weit abgebaut werden, bis sie zu 80 bis 100 Gewichtsprozent bei einer Temperatur von 0 - 40 Grad Celsius in physiologischer Kochsalzlösung löslich sind. Das geschieht durch Abstoppen der Proteaseaktivität durch geeignete Inhibitoren.

Aus dem gleichen Grunde ist es zweckmäßig, die IF-Proteine nur so weit abzubauen, daß bei mindestens 80 Gewichtsprozent das alpha-helikale Mittelstück intakt bleibt.

Bevorzugt wird dieses Verfahren angewendet auf Lymphgewebe als Wirtsgewebe, da sich Metastasen im allgemeinen zunächst im Lymphgewebe ansiedeln. Die IF-Proteine dienen dann als "Marker" für das Ursprungsgewebe, das als Fremdgewebe, zum Beispiel als Mammagewebe, in einem Lymphknoten nachgewiesen wurde.

Der teilweise Abbau kann chemisch, zum Beispiel mit Säuren oder Basen erfolgen. Der teilweise Abbau kann auch physikalisch, zum Beispiel durch Erhitzung oder Bestrahlung erfolgen. Vorzugsweise erfolgt der teilweise Abbau jedoch proteolytisch, weil auf diese Weise bei den hier in Frage stehenden Proteinen der Abbau verhältnismäßig leicht genau so zu dosieren ist, daß die Mittelstücke einerseits löslich andererseits für die Identifizierung hinreichend intakt erhalten bleiben.

Nach Zell-Läsion werden nicht lösliche Proteine und Strukturen vielfach proteolytisch abgebaut. Aus IF-Proteinen erhält man nach solchen Abbaureaktionen die relativ stabilen alphahelikalen Mittelstücke, die dann in Körperflüssigkeiten mit immunologischem Verfahren nachgewiesen und bestimmt werden können. Zum Nachweis solcher Läsionen werden die in Blut, Blutserum, Cerebrospinalliquor, Urin, Fruchtwasser, Punktaten oder anderen Körperflüssigkeiten löslich enthaltenen alpha-helikalen Mittelstück-Fragmente immunologisch identifiziert.

In Körperflüssigkeiten können Intermediärfilamentproteine in unlöslicher Form untermischt sein. Damit diese auch bei serologischen Untersuchungen mit in Betracht gezogen werden, wird auf die in Blut, Blutserum,Cerebrospinalliquor, Urin, Fruchtwasser, Punktaten oder anderen Körperflüssigkeiten enthaltenen Proteine abbauend eingewirkt, bis aus eventuell darin vorhandenen, unlöslichen Intermediärfilamentproteine jeweils alpha-helikale Mittelstück-Fragemente freigesetzt sind, dann die lösliche Fraktion abgetrennt und dann werden die in der löslichen Fraktion enthaltenen alpha-helikalen Mittelstück-Fragemente immunologisch identifiziert.

Die Erfindung gestattet es, die Ergebnisse der Bestimmung und Quantifizierung unverzüglich nach Entnahme der Körperflüssigkeit zu ermitteln. Das ist ein besonderer Vorteil.

Die Erfindung gestattet es weiter, den Verlauf pathologischer oder therapeutischer Prozesse - beispielsweise Bestrahlung, Chemotherapie - zu kontrollieren, bei denen Gewebe zerstört wird, indem zur Verlaufskontrolle einer gewebelädierenden Behandlung die Entnahme der Körperflüssigkeit und die Bestimmung und Quantifizierung mehrfach zeitlich nacheinander während der lädierenden Behandlung und/oder danach erfolgt.

Die zur Quantifizierung erforderliche Standardisierung erfolgt zweckmäßig anhand eines definierten Standards, wobei der Standard aus den gereinigten Intermediärfilamentproteinen oder Fragmenten davon gebildet wird.

Vorzugsweise dienen dazu als Standardmaterial Polypeptide, die mit den das Antigen bildenden Abschnitten der Intermediärfilamentproteine identisch sind oder kreuzreagieren und synthetisch oder durch Expression von synthetischer oder rekombinierter DNS in Prokaryonten oder Hefe hergestellt sind.

Die Erfindung wird nun anhand einiger Beispiele näher erläutert.

BEISPIEL 1 Nachweis von Metastasen in Lymphgewebe

a) Es wird zunächst das Feuchtgewicht des Lymphknotengewebes ermittelt. Das Gewebe wird im dreifachen Volumen - bezogen auf das Feuchtgewicht einer phosphatgepufferten Kochsalzlösung (10 mM (millimolar) Natriumphosphat pH 7,4, 150 mM Natrumchlorid) mit Hilfe eines Messerhomogenisators bis zur breiartigen Konsistenz zerkleinert (es wird der Einsatz eines Polytron-Homogenisators der Firma Kinematica, Luzern /Schweiz empfohlen). Dem Gewebebrei wird Chymotrypsin im Verhältnis 1:1000 (berechnet auf das Feuchtgewicht des Gewebes) zugesetzt. Es folgt ein Inkubationsschritt bei 30° C (Grad Celsius) (vorzugsweise im Thermoblock, eventuell im Wasserbad), der nach 60 Minuten abgestoppt wird. Das Abstoppen der Enzymaktivität geschieht durch Zusatz von 5mM NCDC (2-Nitro-4-carboxyphenyl-N-N-diphenylcarbamat). Anschließend wird das Homogenat 30 Minuten bei $10^4$ g (g = Gravitationskonstante) zentrifugiert. Unter diesen Bedingungen werden 80 bis 95 Gewichtsprozent der alpha-helikalen Mittelstücke in noch identifizierbarem Zustand aus den IF-Proteinen freigesetzt.

3

b) Ermittlung des Vimentingehaltes und Bestimmung des Vimentingehaltes

Im zentrifugierten Überstand wird Vimentin immunologisch durch einen Sandwich-ELISA bestimmt. Hierzu dient ein erster monoklonaler Antikörper GP-8 der sogenannte Fänger--Antikörper der gegen ein erstes Epitop des alpha-helikalen Mittelstückes gerichtet ist, und ein zweiter monoklonaler Antikörper V-100, der sogenannte Detektor-Antikörper, der gegen ein zweites Epitop des alpha-helikalen Mittelstückes, das vom ersten Epitop unabhängig und verschieden ist, gerichtet ist. Der Antikörper V - 100 ist mit Peroxidase gekoppelt und wird bei der Bindung an das alpha-helikale Mittelstück über die Enzymaktivität der Peroxidase nachgewiesen. Zur Standardisierung wird bekanntes, gereinigtes alpha-helikales Vimentin-Fragment dem gleichen Sandwich-ELISA mit den gleichen Antikörpern unterworfen. Der so erhaltene Vimentinwert dient dann als Bezugsgröße für die quantitative Auswirkung der Messergebnisse.

c) Ermittlung der Cytokeratine und Bestimmung der Cytokeratingehalte

Im zentrifugierten Überstand werden die vorhandenen Cytokeratine immunologisch durch einen Sandwich-ELISA bestimmt. Hierzu dient ein erster monoklonaler Antikörper PAN-CI, der sogenannte Fänger-Antikörper, der gegen ein erstes Epitop aller Cytokeratine gerichtet ist. Es sind 19 monoklonale Antikörper C-401 bis C-419 vorgesehen, sogenannte Detektorantikörper, die einzeln spezifisch den einzelnen 19 bekannten Cytokeratinen zugeordnet sind. Die Detektorantikörper sind enzymatisch markiert wie die Detektorantikörper V- 100. Der Nachweis und Standardisierung erfolgt entsprechend wie unter b) jedoch immer bezogen auf das spezielle der 19 möglichen Cytokeratine, so daß man den relativen Anteil der einzelnen Cytokeratine berechnen kann.

Wenn nur die Cytokeratine Nr. 5, Nr. 7, Nr. 8, Nr. 14, Nr. 17 und Nr. 19 mit hinreichendem Anteil und gegebenenfalls zusätzlich die Cytokeratine Nr. 15 und Nr. 18 nachgewiesen werden, dann läßt das auf eine Metastase eines Mamma-Tumors schließen.

BEISPIEL 2 Genauere Identifizierung einer Metastase für den Fall einer nach Beispiel 1 gefundenen Mammametastase

Im Falle, daß aufgrund der Untersuchung nach Beispiel 1 b) und c) im axillären Lymphknoten Mammametastasen nachgewiesen wurden, wird der Rezeptor-Gehalt für Östrogen und Progesteron im Homogenat aus Absatz a) Beispiel 1 bestimmt. Diese Rezeptor-Gehaltsbestimmung wird auf den nach Absatz c) Beispiel 1 gewonnen Gehalt an alpha-helikalem Cytokeratin Fragmenten bezogen. Die gewonnene Bezugsgröße ist ein Maß für den Anteil derjenigen Metastasenzellen, die mit dem betreffenden Hormonrezeptor ausgestattet sind. Eine solche Bezugsgröße konnte bislang mit bekannten Mitteln nicht gewonnen werden; sie ist ein Maß für die zweckmäßig einzusetzende Chemotherapie.

BEISPIEL 3 Nachweis von Metastasen in Beckenkammbiopsien

Wie Beispiel 1 mit dem einzigen Unterschied, daß anstelle des Lymphknotengewebes Beckenkammbiopsien eingesetzt wird.

BEISPIEL 4 Nachweis von Metastasen in Sternumpunktaten

Wie Beispiel 1 mit dem einzigen Unterschied, daß anstelle des Lymphknotengewebes Sternumpunktate eingesetzt wird.

BEISPIEL 5 Nachweis von Läsionen im Epithelgewebe zur Differentialdiagnostik

In 1 ml (Milliliter) Serum von Patientenblut werden die vorhandenen Cytokeratine immunologisch durch einen Sandwich-ELISA bestimmt. Hierzu dient ein erster monoklonaler Antikörper PAN-CI, der sogenannte Fänger-Antikörper, der gegen ein erstes Epitop, das in allen im Serum vorhandenen Cytokeratinfragmentkomplexen vorkommt, gerichtet ist. Es sind weitere monoklonale Antikörper C-401 bis C-419 vorgesehen, sogenannte Detektor-Antikörper, die einzeln gegen zweite Epitope gerichtet sind, die spezifisch den einzelnen bekannten 19 Cytokeratinen zugeordnet sind. Das zweite Epitop ist jeweils vom ersten Epitop unabhängig und verschieden. Die Detektor-Antikörper sind enzymatisch markiert mit Peroxidase.

Zur Standardisierung wird das gereinigte alpha-helikale Mittelstück der 19 verschiedenen Cytokeratine dem gleichen Sandwich-ELISA mit den gleichen Antikörpern unterworfen. Die so erhaltenen 19 verschiedenen Cytokeratinwerte dienen dann als Bezugsgrößen für die quantitative Auswertung der Meßergebnisse.

Auf diese Weise ermittelt man den relativen Anteil der einzelnen Cytokeratine. Das läßt eine genaue Spezifizierung des lädierten Epithelgewebes zu und Rückschlüsse auf das Ausmaß der Lädierung.

Wenn so nur die Cytokeratine 8 und 18 nachgewiesen werden, läßt das auf eine Läsion von Hepatozyten, Nierenepithel beziehungsweise von Acini des Pankreas und der von diesen Zelltypen abgeleiteten Tumoren schließen und macht andere Gewebe als Ort der Läsion unwahrscheinlich.

BEISPIEL 6
Nachweis von Läsionen im inneren Epithelialbereich

Wie Beispiel 5, jedoch mit der Beschränkung auf den Fänger-Antikörper PAN-CI und diejenigen Detektor-Antikörper C-408, D-418 und C-419, die den Cytokeratinen Nr. 8, Nr. 18 und Nr. 19 zugeordnet sind. Wenn die Cytokeratine Nr. 8, Nr. 18 und Nr. 19 mit hinreichenden Anteilen nachgewiesen werden, läßt das auf eine Läsion im Epithelialbereich des Darmtraktes schließen und läßt andere Organe als Ort der Läsion unwahrscheinlich erscheinen.

BEISPIEL 7
Frühdiagnostik bei Verdacht auf Neuralrohrdefekte während der Schwangerschaft

10 ml Fruchtwasserpunktat wird zentrifugiert. Das Sediment wird im 3fachen Volumen, bezogen auf das Feuchtgewicht, einer phosphatgepufferten Kochsalzlösung ( 10 mM (millimolar) Natriumphosphat pH 7,4, 150 mM Natriumchlorid) mit Hilfe eines Messerhomogenisators bis zur breiartigen Konsistenz zerkleinert. Dem Homogenat wird Chymotrypsin im Verhältnis 1 : 1000, berechnet auf das Feuchtgewicht des Gewebes, zugesetzt. Es folgt ein Inkubationsschritt bei 30° C im Wasserbad, der nach 60 Minuten abgestoppt wird. Das Abstoppen der Enzymaktivität geschieht durch Zusatz von 5 mM 2-Nitro-4-Carboxyphenyl-N-N-Diphenylcarbamat (NCDC). Anschließend wird das Homogenat 30 Minuten bei $10^4$g (g = Gravitationskonstante) zentrifugiert.

Unter diesen Bedingungen werden 80 bis 95 Gewichtsprozent der alpha-helikalen Mittelstücke in noch identifizierbarem Zustand aus den IF-Proteinen des Homogenats freigesetzt.

Die beiden gewonnenen, zentrifugierten Überstände werden gesondert, jeder für sich, immunologisch auf ihren Gehalt an Neurofilament- und Gliafilamentprotein mit einem Sandwich-ELISA untersucht. Hierzu dient für die beiden Filamenttypen je ein erster monoklonaler Antikörper NF-8 beziehungsweise GL-8, der sogenannte Fänger-Antikörper, der gegen ein jeweils erstes Epitop des alpha-helikalen Mittelstückes der Filamentproteine gerichtet ist. Außerdem dient dazu jeweils ein zweiter monoklonaler Antikörper NF-100 beziehungsweise GL-100, der sogenannte Detektor-Antikörper, der gegen ein zweites Epitop des alphahelikalen Mittelstücks des zugeordneten Filaments gerichtet ist. Das zweite Epitop ist von dem ersten Epitop unabhängig und verschieden.

Die Detektor-Antikörper NF-100 beziehungsweise GL-100 sind mit Peroxidase gekoppelt und werden bei der Bindung an das alpha-helikale Mittelstück über die Enzymaktiviät der Peroxidase nachgewiesen. Die Standardisierung erfolgt entsprechend wie im Beispiel 5 angegeben.

Das Auftreten von Glia- beziehungsweise Neurofilamentfragmenten in Fruchtwasserproben weist auf Neuralrohrdefekte hin und liefert so einen wesentlich verläßlicheren und schnelleren pränataldiagnostischen Test als bisher benutzte bekannte Methoden.

BEISPIEL 8 bis 10

Wie Beispiel 5 bis 7, wobei jedoch statt den einzelnen Cytokeratinen einzeln zugeordnete Fänger-Antikörper solche Fänger-Antikörper eingesetzt werden, die in Kombination die einzelnen Cytokeratine erkennen. Zum Beispiel die Kombination C-501 und C-504 erkennt das Cytokeratin Nr. X, die Kombination C-501 und C-503 erkennt das Cytokeratin (X + 1).

BEISPIEL 11 bis 20

Wie Beispiel 1 bis 10 mit dem einzigen Unterschied, daß als Standardmaterial für die Standardisierung Peptide dienen, die mit den das Antigen bildenden Abschnitten aus dem alphahelikalen Mitelstück identisch sind und die, ausgehend von cDNS-Sequenzdaten als Quelle für bestimmte Konstrukte und Deletionskonstrukte gentechnologisch gewonnen sind.

EP 0 267 355 B1

## Patentansprüche

1. Verfahren zum Identifizieren gewebstypischer, unlöslicher Intermediärfilamentproteine mit spezifischen Antikörpern, deren Antigene gewebstypische Abschnitte der Intermediärfilamentproteine darstellen, dadurch gekennzeichnet,

   daß die alpha-helikalen Mittelstücke oder Teile dieser Mittelstücke, die das Antigen tragen und die den charakteristischen Abschnitten entsprechen, in Lösung gebracht und dann immunologisch bestimmt und quantifiziert werden.

2. Verfahren nach Anspruch 1, dadurch gekennzeichnet,

   daß aus Zellgewebe ein Homogenat gebildet wird,

   daß auf das Homogenat abbauend eingewirkt wird, bis aus eventuell dann enthaltenen Intermediärfilamentproteinen die alpha-helikale Mittelstückfragmente freigesetzt sind,

   daß dann die lösliche Fraktion abgetrennt wird und,

   daß dann die in der löslichen Fraktion enthaltenen alpha-helikalen Mittelstück-Fragmente in löslicher Phase immunologisch identifiziert werden.

3. Verfahren nach Anspruch 2, dadurch gekennzeichnet,

   daß verschiedene Intermediärfilamentproteine eines Zellhomogenats identifiziert und mengenmäßig zueinander ins Verhältnis gesetzt werden.

4. Verfahren nach Anspruch 1, dadurch gekennzeichnet,

   daß die in Blut, Blutserum, Cerebrospinalliquor, Urin, Fruchtwasser, Punktaten oder anderen Körperflüssigkeiten löslich enthaltenen alpha-helikalen Mittelstück-Fragmente immunologisch identifiziert werden.

5. Verfahren nach Anspruch 4, dadurch gekennzeichnet,

   daß auf die in Blut, Blutserum, Cerebrospinalliquor, Urin, Fruchtwasser, Punktaten oder anderen Körperflüssigkeiten enthaltenen Proteine abbauend eingewirkt wird, bis aus eventuell darin vorhandenen, unlöslichen Intermediärfilamentproteine jeweils alphahelikale Mittelstück-Fragemente freigesetzt sind,

   daß dann die lösliche Fraktion abgetrennt wird und

   daß dann die in der löslichen Fraktion enthaltenen alpha-helikalen Mittelstück-Fragmente immunologisch identifiziert werden.

6. Verfahren nach Anspruch 1, dadurch gekennzeichnet,

   daß die Quantifikation anhand eines definierten Standards erfolgt, wobei der Standard aus den gereinigten Intermediärfilamentproteinen oder Fragmenten davon gebildet wird.

7. Verfahren nach Anspruch 6, dadurch gekennzeichnet,

   daß als Standardmaterial Polypeptide dienen, die mit den das Antigen bildenden Abschnitten der Intermediärfilamentproteine identisch sind oder kreuzreagieren und synthetisch oder durch Expression von synthetischer oder rekombinierter DNS in Prokaryonten oder Hefe hergestellt sind.

## Claims

1. Process for identifying tissue-specific, insoluble intermediate filament proteins with specific antibodies, whose antigens constitute tissue-specific portions of the intermediate filament proteins, characterized in that the alpha-helical central portions or parts of said central portion carrying the antigen and which corresponds to the characteristic portions are dissolved and then immunologically determined and quantified.

2. Process according to claim 1, characterized in that a homogenate is formed from cellular tissue, a decomposing action is brought about on the homogenate until the alpha-helical central portion fragments are released from any intermediate filament proteins contained therein, that the soluble fraction is separated and that then the alpha-helical central portion fragments contained in the soluble fraction are immunologically identified in the soluble phase.

6

3. Process according to claim 2, characterized in that different intermediate filament proteins of a cellular homogenate are identified and are brought into a quantitative ratio with one another

4. Process according to claim 1, characterized in that the alphahelical central portion fragments contained in soluble form in blood, blood serum, cerebrospinal liquor, urine, amniotic fluid, punctates or other body fluids are immunologically identified. ·

5. Process according to claim 4, characterized in that a decomposing action is brought about on the proteins contained in the blood, blood serum, cerebrospinal liquor, urine, amniotic fluid, punctates or other body fluids until alpha-helical central portion fragments are released from any insoluble intermediate filament proteins present therein, that then the soluble fraction is separated and that then the alpha-helical central portion fragments contained in the soluble fraction are immunologically identified.

6. Process according to claim 1, characterized in that the quantification takes place by means of a specific standard, the standard being formed from the purified intermediate filament proteins or fragments thereof.

7. Process according to claim 6, characterized in that polypeptides are used as the standard material and are identical or cross-react with the portions of the intermediate filament proteins forming the antigen and are prepared synthetically or by the expression of synthetic or recombined DNA in prokaryons or yeast.

**Revendications**

1. Procédé d'identification de protéines fibreuses ou (micro)filamentaires intermédiaires insolubles, typiques de tissus, au moyen d'anticorps spécifiques, dont les antigènes représentent des tronçons, typi - ques de tissus, de protéines fibreuses intermédiaires, procédé caractérisé en ce qu'on met en solution les pièces médianes d'hélice alpha, ou des parties de ces pièces médianes, qui portent l'antigène et qui correspondent aux tronçons caractéristiques, et on les soumet ensuite à détermination immunologique et à quantification.

2. Procédé selon la revendication 1, caractérisé en ce qu'on forme un homogénéisat de tissu de cellules; on exerce sur l'homogénéisat des effets de dégradation jusqu'à mettre en liberté, à partir de protéines fibreuses intermédiaires éventuellement contenues, les fragments de pièces médianes d'hélice alpha; on sépare ensuite la fraction soluble, et l'on soumet ensuite à identification immunologique, dans la phase soluble, les fragments de pièces médianes d'hélice alpha contenus dans la fraction soluble.

3. Procédé selon la revendication 2, caractérisé en ce qu'on identifie les diverses protéines fibreuses intermédiaires d'un homogénéisat de cellules et l'on en établit le rapport quantitatif mutuel.

4. Procédé selon la revendication 1, caractérisé en ce qu'on soumet à identification immunologique les fragments de pièces médianes d'hélice alpha contenus en dissolution dans le sang, le sérum le liquide céphalo-rachidien, l'urine, le liquide ou eau amniotique, des produits de ponction ou d'autres liquides corporels.

5. Procédé selon la revendication 4, caractérisé en ce qu'on soumet les protéines contenues dans le sang, le sérum sanguin, le liquide cérébro-spinal (ou céphalo-rachidien), le liquide ou eau amniotique, des produits de ponction et d'autres liquides corporels à des effets de dégradation, exercés jusqu'à mise en liberté, à partir de protéines fibreuses intermédiaires insolubles qui y sont éventuellement contenues, à chaque fois des fragments de pièces médianes d'hélice alpha; on sépare ensuite la fraction soluble; puis l'on soumet à identification immunologique les fragments de pièces médianes d'hélice alpha contenus dans la fraction soluble.

6. Procédé selon la revendication 1, caractérisé en ce qu'on effectue la quantification en regard d'un témoin standard bien défini, le témoin étant formé de protéines fibreuses intermédiaires purifiées ou de fragments de ces protéines.

7. Procédé selon la revendication 6, caractérisé en ce que servent de matière témoin des polypeptides, qui sont identiques aux tronçons de protéines fibreuses intermédiaires formant l'antigène, ou effectuent avec eux des réactions croisées, et qui ont été obtenus par synthèse ou par expression d'acide désoxyribonucléique ( ADN ), synthétique ou recombiné, dans des procaryotes ou des levures.